(19) Europäisches Patentamt
European Patent Office
Office européen des brevets

(11) Numéro de publication: **0 103 500**
**B1**

(12) # FASCICULE DE BREVET EUROPÉEN

(45) Date de publication du fascicule du brevet: **13.01.88**

(21) Numéro de dépôt: **83401613.1**

(22) Date de dépôt: **04.08.83**

(51) Int. Cl.⁴: **C 07 D 211/34**, A 61 K 31/445, C 07 D 405/06 // C07D211/18, C07D211/22, C07D211/60, C07D317/64

(54) **Dérivés de phénéthyl-1alpha-phényl-pipéridine-3-propanenitrile, leur préparation et leur application en thérapeutique.**

(30) Priorité: **16.08.82 FR 8214154**

(43) Date de publication de la demande: **21.03.84 Bulletin 84/12**

(45) Mention de la délivrance du brevet: **13.01.88 Bulletin 88/2**

(84) Etats contractants désignés: **AT BE CH DE FR GB IT LI LU NL SE**

(56) Documents cité:
**DE-A-1 445 427**
**FR-A-2 302 739**
**GB-A-1 057 145**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Manoury, Philippe, 9, rue de Malabry, F-92350 Le Plessis- Robinson (FR)**
Inventeur: **Binet, Jean, 12, hameau de la Gondole, F-91650 Breuillet (FR)**
Inventeur: **Defosse, Gérard, 8, Place Verlaine, F-75013 Paris (FR)**

(74) Mandataire: **Thouret- Lemaitre, Elisabeth, Service Brevets - SYNTHELABO 58, rue de la Glacière, F-75621 Paris Cédex 13 (FR)**

EP 0 103 500 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne des dérivés hétérocycliques, leur préparation et leur application en thérapeutique. Les composés de l'invention répondent à la formule suivante (I)

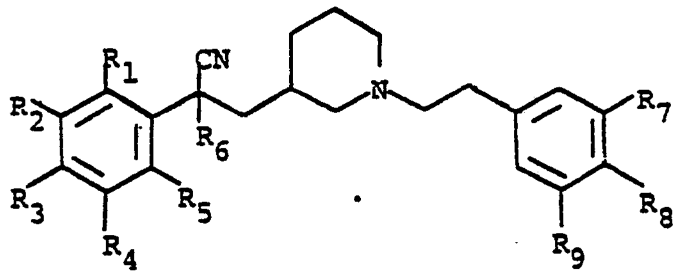

(I)

dans laquelle

· $R_1$, $R_2$, $R_3$, $R_4$ et $R_5$, représentent chacun, indépendamment l'un de l'autre, un atome d'hydrogène, un atome d'halogène, un radical ($C_{1-4}$) alcoxy, un radical ($C_{1-4}$) alkylthio, un radical halogéno-($C_{1-4}$) alkyle, un radical ($C_{1-4}$) alkyle, un radical cycloprofylméthoxyéthoxy, ou deux des radicaux forment ensemble un radical méthylènedioxy ou éthylènedioxy,

· $R_6$ représente un atome d'hydrogène, un radical ($C_{1-4}$) alkyle droit ou ramifié, un radical ($C_{3-6}$) cycloalkyle ou un radical ($C_{3-6}$) cycloalkyl-($C_{1-4}$)alkyle, et

· $R_7$, $R_8$ et $R_9$ représentent chacun, indépendamment l'un de l'autre, un atome d'hydrogène ou un radical méthoxy.

Les sels d'addition à des acides pharmaceutiquement acceptables que forment les composés font partie de l'invention.

Les composés de l'invention comportent deux carbones asymétriques et peuvent fournir par conséquent deux couples de diastéréoisomères que l'on sépare par cristallisation fractionnée de leurs sels ou par chromatographie.

Le couple de diastéréoisomères A désigne le produit dont le Rf en CCM est inférieur à celui du couple de diastéréoisomères B.

L'oxalate du couple de diastéréoisomères A est plus insoluble que celui du couple de diastéréoisomères B. Les spectres RMN des couples de diastéréoisomères A et B sont différents et permettent de les identifier.

Les énantiomères de chaque couple de diastéréoisomères font également partie de l'invention et peuvent être obtenus

- soit par cristallisation fractionnée de sels d'acides optiquement actifs,
- soit par synthèse à partir d'un composè (III) dont l'un des atomes de carbone du noyau pipéridinique a une stéréochimie connue; cette synthèse conduit à deux diastéréoisomères de configuration RS et SS ou RR et RS selon la stéréochimie du carbone du noyau pipéridinique; ces diastéréoisomères sont séparables par cristallisation fractionnée de leurs sels ou par chromatographie.

Les composés préférés de l'invention sont ceux pour lesquels deux des radicaux $R_1$ à $R_5$ sont différents de H, et plus particulièrement ceux pour lesquels $R_1$ à $R_5$ représentent chacun, indépendamment l'un de l'autre, un radical méthoxy, méthylthio, méthyle, ou cyclopropylméthoxy-2 éthoxy, ou un atome d'hydrogène, de chlore ou de fluor, ou deux des radicaux $R_1$ à $R_5$ représentent ensemble un radical méthylènedioxy ou éthylènedioxy.

Parmi ces composés ceux pour lesquels $R_6$ est un radical isopropyle, éthyle, cyclopentyle ou cyclopropylméthyle ou un atome d'hydrogène sont préférés et tout particulièrement ceux pour lesquels $R_6$ est un radical isopropyle.

Un ensemble de composés particulièrement préférés est celui de formule générale (I) dans laquelle deux des radicaux $R_1$ à $R_5$ représentent un radical méthoxy, ou le radical méthylènedioxy ou éthylènedioxy et un autre des radicaux $R_1$ à $R_5$ représente un atome d'hydrogène ou le radical méthoxy, $R_6$ est le radical isopropyle, $R_7$ et $R_8$ représentent chacun un radical méthoxy, et $R_9$ est un atome d'hydrogène.

Des composés analogues à ceux de l'invention sont décrits dans le brevet français n° 2 302 739 de la demanderesse, comme antiarythmisants et antiangineux mais, contrairement à ceux de l'invention, ils ne sont pas anticalciques ni antihypertenseurs. D'autres composés, encore bien plus différents, sont connus grâce au brevet allemand n° 1 445 427, comme analgésiques, ou grâce au brevet britannique n° 1 057 145, comme antitussifs et spasmolytiques.

Selon l'invention on peut préparer les composés (I) selon le schéma réactionnel suivant

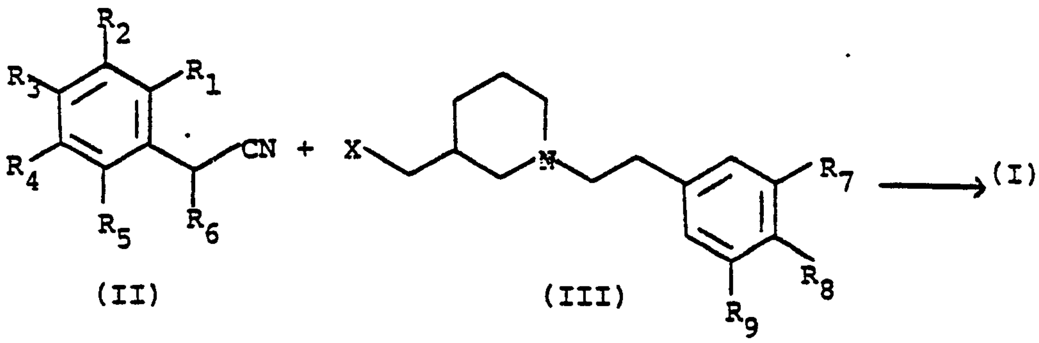

(II) (III)

X est un groupe labile, tel qu'un atome d'halogène ou un radical alkylsulfonyle ou arylsulfonyle.

Les composés de départ (II) sont en partie nouveaux et en partie décrits dans la littérature. Ils sont préparés selon des méthodes décrites dans la littérature, par réaction entre un composé

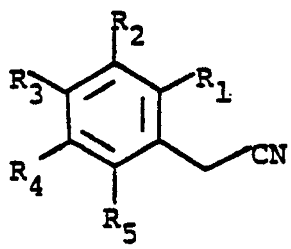

et un composé $R_6$ Y (Y = groupe labile tel que X)

- dans du diméthylsulfoxyde en présence de soude,
- dans du diméthylformamide en présence d'hydrure de sodium,
- dans du toluène en présence d'amidure de sodium.

Les composés de départ (III) sont nouveaux et sont préparés selon les schémas réactionnels suivants.

**Schema 1**

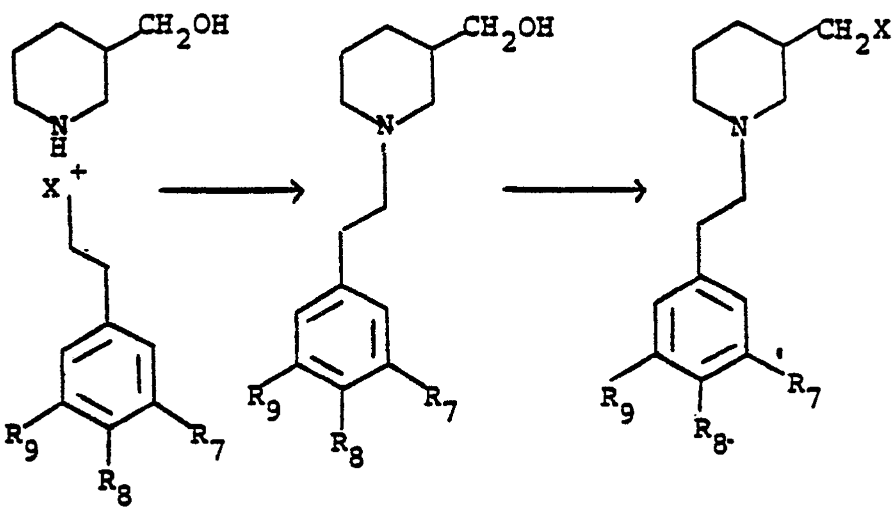

**Schema 2**

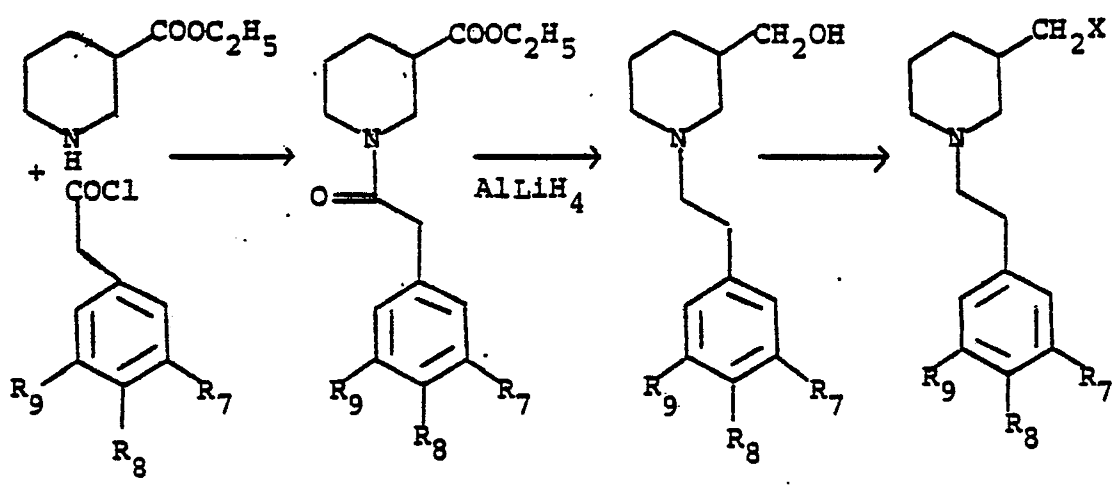


**Schema 3**

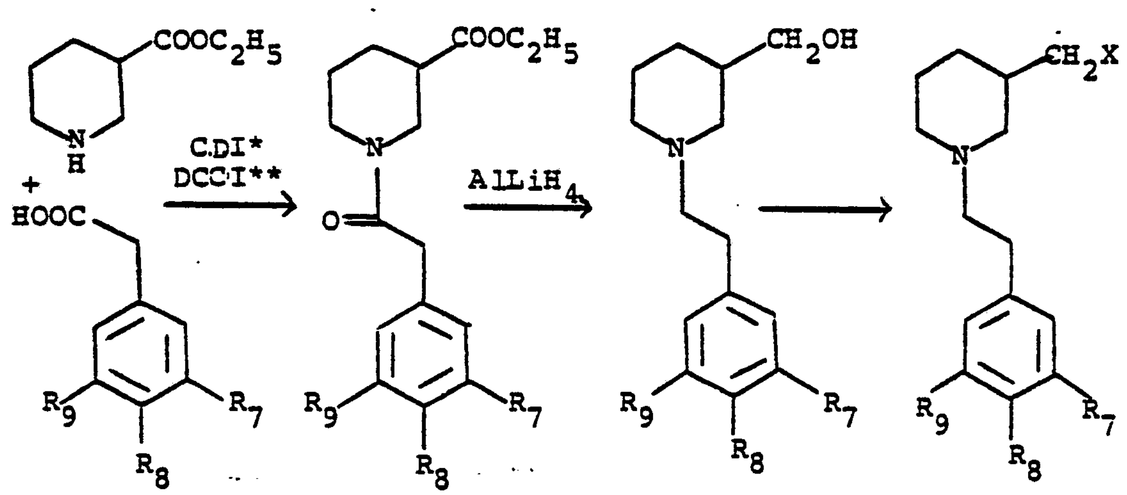


* CDI = carbonyldiimidazole ** DCCI = dicyclohexylcarbodiimide

La réaction entre le composé (II) et le composé (III) est effectuée

- soit dans du toluène en présence d'amidure de sodium, à une température de 60 à 110°C,
- soit dans un mélange de tétrahydrofuranne et d'hexaméthylphosphorotriamide (HMPT) en présence de diisopropylamidure de lithium à une température de -60 à + 20°C,
- soit par transfert de phase avec une base minérale, telle que la soude et un solvant tel que le toluène ou le chlorure de méthylène en présence d'un ammonium quaternaire tel que le chlorure ou le bromure de tétrabutylammonium, à une température de 20 à 100°C.

Les exemples suivants illustrent l'invention. Les analyses et les spectres IR et RMN confirment la structure des composés.

**Exemple 1**

α-(dichloro-3,4 phényl) [(diméthoxy-3,4 phényl)-2 éthyl]-1 α-(méthyl-1 éthyl)-pipéridine-3-propane-nitrile.

1.1. [(diméthoxy-3,4 phényl)-éthyl] -1 hydroxyméthyl-3 pipéridine).

On porte à la température du reflux pendant une journée un mélange de 16 g (0,08 mole) de chlorure de diméthoxy-3,4 phényléthyle, de 9,2 g (0,08 mole) d'hydroxyméthyl-3 pipéridine, de 2 g d'iodure de sodium, de 17 g de carbonate de sodium et de 50 cm$^3$ de méthyléthylcétone. On évapore le solvant et reprend le résidu par un mélange de soude diluée et d'éther. On lave la phase éthérée 3 fois avec de l'eau, on sèche sur $Na_2SO_4$ et évapore.

Après recristallisation dans de l'éther isopropylique, la [(diméthoxy-3,4 phényl)-éthyl]-1 hydroxyméthyl-3 pipéridine fond à 89°C.

On prépare le chlorhydrate de ce composé dans de l'acétone en faisant réagir la base avec de l'éther chlorhydrique. Il fond à 167° C.

1.2. [(diméthoxy-3,4phényl)-2éthyl]-1 chlorométhyl-3pipéridine

On agite à la température ambiante pendant 2 heures un mélange de 4,8 g (0,0152 mole) du chlorhydrate obtenu sous 1.1, de 5,4 g (0,045 mole) de $SOCl_2$ et de 50 $cm^3$ de chloroforme. On porte ce mélange à la température de reflux pendant 1 heure. On évapore le solvant sous vide puis reprend le résidu par du toluène. Après évaporation le chlorhydrate de [(diméthoxy-3,4 phényl)-2éthyl]-1 chlorométhyl-3 pipéridine cristallise.

Après lavage à l'acétone et à l'éther ce composé fond à 171° C.

On obtient la base par addition d'eau carbonatée et d'éther, sous forme d'huile.

1.3. α-(dichloro-3,4 phényl) [(diméthoxy-3,4 phényl)-2 éthyl)] -1 α-(méthyl-1 éthyl)-pipéridine-3-propanenitrile.

On porte à la température de 70° C, pendant une demi-heure, un mélange de 5 g (0,0219 mole) de (dichloro-3,4 phényl)-2 isopropyl-2 acétonitrile, de 6 g (0,02 mole) de [(diméthoxy-3,4 phényl)-2 éthyl]-1 chlorométhyl-3 pipéridine et de 1,96 g (0,024 mole) de $NaNH_2$ en suspension à 50 % dans du toluène et 40 $cm^3$ de toluène.

On chauffe progressivement à la température du reflux pendant 2 heures. On refroidit le mélange réactionnel, on extrait à l'éther et verse sur de l'eau glacée. Après les traitements habituels le produit est extrait à l'éther, lavé et séché. On obtient une huile que l'on transforme en oxalate. On obtient alors un précipité qui, après recristallisation dans du méthanol, donne le couple de diastéréoisomères A sous forme d'oxalate (F=208° C) que l'on transforme en base puis en chlorhydrate (F=220° C).

A partir du filtrat on récupère l'autre couple de diastéréoisomères B par alcalinisation. On obtient une huile que l'on transforme en fumarate acide (F=176° C) dans un mélange alcool/éther.

On libère la base puis prépare le chlorhydrate (F=201° C).

**Exemple 2**

[(diméthoxy-3,4 phényl)-2 éthyl] -1 α-(diméthoxy-3,4 phényl) α-(méthyl-1 éthyl)-pipéridine-3-propane-nitrile.

On porte à la température du reflux, pendant 8 heures, un mélange de 3,5 g (0,0117 mole) de [(diméthoxy-3,4 phényl)-2éthyl]-1 chlorométhyl-3 pipéridine, de 2,7 g (0,0123 mole) de (diméthoxy-3,4 phényl)-2 isopropyl-2 acétonitrile, de 1,09 g (0,014 mole) de $NaNH_2$ en suspension à 50 % dans du toluène et 30 $cm^3$ de toluène.

Après 6 heures de reflux, on ajoute à nouveau 0,1 g du nitrile et 0,04 g de $NaNH_2$ à 50 %.

Après évaporation sous vide et reprise par un mélange d'eau et d'éther, on sèche et on évapore. On transforme l'huile obtenue en oxalate acide. Par cristallisation on obtient l'un des deux couples de diastéréoisomères. Après recristallisation dans du méthanol, le couple de diastéréoisomères A sous forme d'oxalate fond à 203° C. On le transforme en base puis en fumarate plus soluble qui fond à 105° C.

Le couple de diastéréoisomères B, sous forme d'oxalate obtenu à partir du filtrat et après recristallisation, fond à 179° C.

**Exemple 3**

[(diméthoxy-3,4 phényl)-2 éthyl]-1 α-(triméthoxy-3,4,5 phényl) α-(méthyl-1 éthyl)-pipéridine-3-propanenitrile.

On porte à la température du reflux sous azote, pendant 16 heures, un mélange de 5,3 g (0,0178 mole) de [(diméthoxy-3,4 phényl)-2 éthyl]-1 chlorométhyl-3 pipéridine, de 4,7 g (0,019 mole) de (triméthoxy-3,4,5 phényl)-2 isopropyl-2 acétonitrile, de 1,7 g (0,0212 mole) de $NaNH_2$ à 50 % en suspension dans du toluène et de 40 $cm^3$ de toluène. Au cours de la réaction on ajoute un excès de nitrile et la quantité équimolaire de $NaNH_2$ jusqu'à disparition complète du composé chloré de départ, en poursuivant le reflux.

Après les traitements habituels de lavage et de séchage on récupère une huile que l'on transforme en oxalate dans de l'éthanol.

Les deux couples de diastéréoisomères sont séparés par chromatographie et transformés en sels.

Le couple de diastéréoisomères A sous forme de fumarate acide fond à 146° C.

Le couple de diastéréoisomères B sous forme de chlorhydrate fond à 192° C.

**Exemple 4**

[(diméthoxy-3,4 phényl)-2 éthyl]-1 α-(méthoxy-2 méthylène-dioxy-3,4 phényl) α-(méthyl-1 éthyl)-pipéridine-3-propanenitrile.

1. (Méthoxy-2 méthylènedioxy-3,4 phényl)-2 isopropyl-2 acétonitrile.

Le (méthoxy-2 méthylènedioxy-3,4 phényl)-2 acétonitrile de départ est décrit dans la littérature, par A. Robertson et W. B. Whalley, J. Chem. Soc. 1440-(1954).

On introduit 6,5 g (0,034 mole) de ce nitrile en solution dans 20 $cm^3$ de DMF, dans une suspension de 1,7 g

(0,036 mole) de NaH (à 50 % dans l'huile) dans 100 $cm^3$ de DMF.

Lorsque l'addition est terminée on ajoute 3,4 $cm^3$ (0,036 mole) de bromo-2 propane et agite le mélange une demi-heure à la température ambiante. On verse le mélange réactionnel sur 200 $cm^3$ d'eau glacée et extrait 2 fois avec de l'éther.

On lave la phase éthérée avec de l'eau, sèche, filtre et évapore. On purifie le produit par chromatographie sur colonne (éluant $CH_2Cl_2$) et on recueille une huile jaune pâle.

2. La [(diméthoxy-3,4 phényl)-2 éthyl]-1 chlorométhyl-3 pipéridine est préparée selon le procédé de l'exemple 1.

3. [(diméthoxy-3,4 phényl)-2 éthyl] -1 α-(méthoxy-2 méthylène-dioxy-3,4 phényl) α-(méthyl-1 éthyl)-pipéridine-3-propanenitrile.

On refroidit à -60°C une solution de 50 $cm^3$ de THF et de 50 $cm^3$ de HMPT contenant 1,82 $cm^3$ (0,025 mole) de diisopropylamine.

On ajoute sous argon, à -60°C, 10,6 $cm^3$ (0,025 mole) de BuLi (1,6 mole dans l'hexane). On agite le mélange 15 mn et ajoute 4,7 g (0,02 mole) du nitrile préparé sous 1 en solution dans 40 $cm^3$ de THF. On agite une demi-heure en laissant la température revenir à -40°C, puis refroidit le mélange à -60°C et ajoute une solution de 4,5 g (0,015 mole) de [(diméthoxy-3,4 phényl)-2 éthyl)] -1 chlorométhyl-3 pipéridine dans 40 $cm^3$ de THF.

On laisse le mélange revenir à la température ambiante, puis le laisse au repos 48 h. On verse le mélange sur de l'eau glacée et extrait 2 fois avec de l'éther.

On lave la phase éthérée avec de l'eau, sèche, filtre et évapore.

On chromatographie l'huile résiduelle sur une colonne de silice.

On prépare l'oxalate des 2 couples de diastéréoisomères A et B dans du méthanol. Après recristallisation dans du méthanol on obtient l'oxalate du couple de diastéréoisomères A fondant à 238°C.

On évapore les eaux mères de cristallisation. On reprend par de l'eau, alcalinise, extrait avec $CH_2Cl_2$, sèche, évapore. On obtient ainsi la base sous sous la forme d'une huile que l'on transforme en chlorhydrate dans un mélange acétate d'éthyle/éther.

Ce produit est constitué par le chlorhydrate du couple de diastéréoisomères B fondant à 210-211°C.

**Exemple 5**

[(diméthoxy-3,5 phényl)-2 éthyl]-1 α-(diméthoxy-3,5 phényl) α-(méthyl-1 éthyl)- pipéridine-3-propanenitrile.

1. (Diméthoxy-3,5 phényl)-2 isopropyl-2 acétonitrile.

Il est préparé par réaction entre le (diméthoxy-3,5 phényl)-2 acétonitrile (4 g, 0,022 mole) en solution dans 10 $cm^3$ de DMF avec 2,86 g de bromure d'isopropyle dans 5 $cm^3$ de DMF, en présence de 1,1 g d'hydrure de sodium à 50 % dans 25 $cm^3$ de DMF.

$Eb_{0,85} = 124°C$

2. [(Diméthoxy-3,5 phényl)-2 éthyl] -1 chlorométhyl-3 pipéridine.

· On fait réagir 25 g d'acide (diméthoxy-3,5 phényl)-2 acétique dans 100 $cm^3$ de THF, 23 g de carbonyldiimidazole dans 200 $cm^3$ de THF et 20 g de pipéridine-3 carboxylate d'éthyle, à une température de 15°C.

· On réduit le [(diméthoxy-3,5 phényl)-méthyl-carbonyl]-1 pipéridine-3-carboxylate d'éthyle (38,5 g; 0,115 mole) en solution dans 100 $cm^3$ de THF avec 8 g de $LiAlH_4$ en solution dans 100 $cm^3$ de THF.

On obtient une huile jaune pâle que l'on transforme en chlorhydrate.

· On transforme le chlorhydrate de [(diméthoxy-3,5 phényl)-2 éthyl] -1 pipéridine-3-méthanol (8 g) en solution dans 50 $cm^3$ de $CHCl_3$, en [(diméthoxy-3,5 phenyl)-2 éthyl] -1 chlorométhyl-3 pipéridine à l'aide de 6 $cm^3$ de $SOCl_2$ en solution dans 10 $cm^3$ de $CHCl_3$, sous azote.

3. [(diméthoxy-3,5 phényl)-2 éthyl]-1 α-(diméthoxy-3,5 phényl) α-(méthyl-1 éthyl)-pipéridine-3-propanenitrile.

On refroidit à -50°C une solution de 1,8 g (0,0175 mole) de diisopropylamine dans 50 $cm^3$ de THF et 50 $cm^3$ de HMPT. On ajoute goutte à goutte une solution de 11 $cm^3$ (0,0175 mole) de butyl-lithium 1,6 M.

On agite le mélange réactionnel à -50°C pendant 20 mn puis on ajoute la solution de 2,75 g (0,0125 mole) du nitrile préparé sous 1 dans 10 $cm^3$ de THF, goutte à goutte, on agite à nouveau à -50°C pendant 30 mn puis on ajoute la solution de 3 g du compose préparé sous 2 dans 10 $cm^3$ de THF. On agite le mélange réactionnel pendant 1 h à -50°C et le laisse reposer une nuit à la température ambiante. On le verse dans 0,5 l d'un mélange eau/glace, on le sature avec NaCl et l'extrait par de l'éther. On sèche et évapore la phase organique. On chromatographie l'huile obtenue sur une colonne de silice 60 en éluant avec un mélange 70/30 $CH_2Cl_2$/acétone.

Après évaporation des solvants, on met l'huile en solution dans de l'éthanol et ajoute de l'acide oxalique en quantité stoechiométrique. On filtre le couple de diastéréoisomères A, le lave avec de l'éthanol et de l'éther.

F=210°C.

On évapore à siccité les eaux mères de cristallisation puis reprend le résidu par un mélange $CH_2Cl_2/NaHCO_3$, on laisse décanter la phase organique, la sèche et l'évapore. On chromatographie la solution, ajoute de l'acide oxalique et obtient l'oxalate du couple de diastéréoisomères B.

F=150°C.

**Exemple 6**

Enantiomères $A_1$, $A_2$, $B_1$ et $B_2$ du [(diméthoxy-3,4 phényl)-2 éthyl] -1 α-(diméthoxy-3,4 phényl) α-(méthyl-1 éthyl)-pipéridine-3-propanenitrile.

6.1. (R)(-) et (S)(+) pipéridine-3-carboxylates d'éthyle.

On abandonne pendant 4 jours à 0-5°C une solution de 200 g (1,27 mole) de pipéridine-3-carboxylate d'éthyle et de 191 g (1,27 mole) d'acide L (+) tartrique dans 800 cm³ d'éthanol. On filtre le précipité formé et le sèche. Après 2 recristallisations dans de l'éthanol on obtient 128 g d'un produit fondant à 153°C de configuration (R).

$[\alpha]^{20}_D = +10{,}42°$ (c=5; $H_2O$)

On évapore à sec les eaux mères, reprend le résidu d'évaporation avec une petite quantité d'eau, alcalinise avec un léger excès de soude concentrée et extrait 2 fois avec de l'éther. On sèche la phase éthérée avec $MgSO_4$, filtre, évapore et distille.

On obtient 73 g (0,46 mole) d'une huile bouillant à 103°C sous un vide de 7 mm de mercure. On solubilise cette huile dans 450 cm³ d'éthanol contenant 70 g (0,46 mole) d'acide D(-)tartrique.

On laisse au repos et obtient après filtration 111 g de sel que l'on recristallise dans 800 cm³ d'éthanol contenant 10 cm³ d'eau. Le produit de configuration (S) fond à 154,5°C.

$[\alpha]^{20}_D = -10{,}1°$ (c=5;$H_2O$)

Les tartrates sont convertis en bases par alcalinisation avec de la soude et extraction avec de l'éther.

On obtient ainsi

59 g de (R)(-)pipéridine-3-carboxylate d'éthyle

$[\alpha]^{20}_D = -1{,}9°$ (c=5; $H_2O$)

et 45,6 g de (S)(+) pipéridine-3-carboxylate d'éthyle

$[\alpha]^{20}_D = +2°$ (c=5; $H_2O$)

6.2. [(diméthoxy-3,4 phényl)-2 éthyl]-1 α-(diméthoxy-3,4 phényl) α-(méthyl-1 éthyl)- pipéridine- 3,*S*)-3-propanenitrile.

6.2.1. (+) hydroxyméthyl-3 pipéridine-(3,*R*).

A une suspension de 6,5 g (0,17 mole) de $AlLiH_4$ dans 100 cm³ d'éther sec, on ajoute goutte à goutte 20,1 g (0,128 mole) de (R)(-) pipéridine-3-carboxylate d'éthyle en solution dans 50 cm³ d'éther de telle façon que l'éther soit légèrement au reflux.

Après la fin de l'addition on maintient le reflux 1 h. On refroidit le mélange et détruit le complexe avec précaution avec un mélange eau-acétate d'éthyle. On filtre, rince avec de l'éther puis évapore le filtrat. On obtient 8,8 g (60 %) d'un produit cristallisé fondant à 62°C (Eb=126°C sous 10 mm de Hg) $[\alpha]^{20}_D = +2{,}9°$ (c=0,6; pyridine).

6.2.2. (+)[(Diméthoxy-3,4 phényl)-2 éthyl] -1 hydroxyméthyl-3 pipéridine-(3,*R*).

On porte à la température du reflux, pendant 60 heures un mélange de 8,6 g (0,075 mole) de (+) hydroxyméthyl-3 pipéridine-(3,*R*), 16,5 g (0,082 mole) de chlorure de (diméthoxy-3,4 phényl)-2 éthyle, de 3 g de NaI et de 17 g (0,16 mole) de $Na_2CO_3$ dans 50 cm³ de méthyléthylcétone.

On évapore le mélange réactionnel, reprend le résidu par un mélange eau-éther et filtre l'insoluble. On lave la phase organique avec de l'eau, sèche avec $Na_2SO_4$, filtre et évapore. On prépare le chlorhydrate du composé dans un mélange acétone/éther. On obtient un produit fondant à 188°C.

$[\alpha]^{20}_D = +8{,}4°$ (c=1 dans $CH_3OH$)

6.2.3. (+) [(diméthoxy-3,4 phényl) éthyl] -1 chlorométhyl-3 pipéridine-(3,*R*)

On ajoute goutte à goutte, en refroidissant à 5°C, 12,5 cm³ (0,17 mole) de $SOCl_2$ dans 60 cm³ de chloroforme à une solution de 18 g (0,057 mole) de chlorhydrate de (+) [(diméthoxy-3,4 phényl)-2 éthyl] -1 hydroxyméthyl-3 pipéridine-(3,*R*) dans 200 cm³ de $CHCl_3$.

On laisse la solution revenir à la température ambiante en 2 h environ puis la porte à la température du reflux 1 h 30. On refroidit le mélange, évapore. On dissout le résidu d'évaporation dans 50 cm³ de méthanol, ajoute 100 cm³ d'éther et laisse cristalliser lentement. On obtient un produit fondant à 202°C

$[\alpha]^{20}_D = +9{,}2°$ (c=1; $CH_3OH$)

6.2.4. [(diméthoxy-3,4 phényl)-2 éthyl] -1 α-(diméthoxy-3,4 phényl) α-(méthyl-1 éthyl)- pipéridine-(3,*S*)-3-propanenitrile.

On chauffe, à la température du reflux, pendant 12 heures, sous azote, un mélange de 12,1 g (0,04 mole) de [(diméthoxy-3,4 phényl)-2 éthyl]-1 chlorométhyl-3 pipéridine-(3,*R*), de 10,1 g (0,046 mole) d'α-(diméthoxy-3,4 phényl) α-isopropyl acétonitrile, de 4,5 cm³ (0,057 mole) d'amidure de sodium (en suspension à 50 % dans du toluène) dans 100 cm³ de toluène.

On refroidit le mélange, le verse sur de l'eau, extrait avec de l'éther. On sèche, filtre et évapore. On chromatographie l'huile résiduelle sur une colonne de silice. On obtient ainsi une huile.

On prépare l'oxalate dans de l'acétate d'éthyle. On obtient ainsi 5 g de produit que l'on reprend avec 300 cm³ d'acétate d'éthyle bouillant. On filtre la suspension bouillante et recristallise l'insoluble dans de l'éthanol. On obtient l'oxalate de l'énantiomère $A_1$ fondant à 172°C.

$[\alpha]^{20}_D = +82{,}2°$ (c=0,5; $CH_3OH$) pour la base

$[\alpha]^{20}_D = +58°$ (c=1; $CH_3OH$) pour l'oxalate

(Rf=0,45 CCM Merck éluant AcO Bu/AcOH/BuOH/$H_2O$:47/28/8,5/16,5)

On reprend le filtrat et ajoute de l'éther; par trituration et refroidissement on obtient l'oxalate de l'énantiomère $B_1$ fondant à 85°C environ.

[α] $^{20}{}_D$ = + 35,5° (c=1; $CH_3OH$)
(Rf=0,5 CCM Merck 5719 éluant AcOBu 47, AcOH 28, BuOH 8,5, $H_2O$ 16,5)

6.3. [(diméthoxy-3,4 phényl)-2 éthyl]-1 α-(diméthoxy-3,4 phényl) α-(méthyl-1 éthyl)-pipéridine-(3,*R*)-3-propanenitrile.

6.3.1. [(diméthoxy-3,4 phényl)-méthylcarbonyl]-1 pipéridine-3-carboxylate d'éthyle.

On refroidit à -20°C une suspension de 28,7 g (0,18 mole) de (S)(+) pipéridine-3 carboxylate d'éthyle dans 200 cm$^3$ de chloroforme et 38,5 à de $Na_2CO_3$.

On ajoute alors goutte à goutte 39,1 g (0,18 mole) de chlorure de diméthoxy-3,4 phénylacétyle en solution dans 100 cm$^3$ de $CHCl_3$. On agite une demi-heure après la fin de l'addition, reprend le mélange avec de l'eau et décante. On lave la phase organique avec de l'eau, sèche, filtre, évapore. On obtient 48 g (79 %) d'huile bouillant à 210°C sous 0,02 mm de Hg.

[α] $^{20}{}_D$ = + 43,3° (c=1; $CH_3OH$)

6.3.2. (-) [(diméthoxy-3,4 phényl)-2 éthyl] -1 pipéridine -(3,*S*)-3-méthanol

A une suspension de 7,7 g (0,21 mole) d'$AlLiH_4$ dans 70 cm$^3$ de THF, on ajoute goutte à goutte 45 g (0,134 mole) de (S)(+) [(diméthoxy-3,4 phényl)-méthylcarbonyl]-1 pipéridine-3-carboxylate d'éthyle en solution dans 200 cm$^3$ de THF. Lorsque l'addition est terminée, on porte le mélange à la température du reflux pendant 1 h 30. On refroidit, hydrolyse le complexe puis filtre. On lave le précipité avec du THF et évapore le filtrat.

On prépare le chlorhydrate dans un mélange éthanol/éther. On obtient ainsi, après recristallisation dans de l'éthanol, un produit fondant à 191°C.

[α] $^{20}{}_D$ = -6,9° (c=1; $CH_3OH$)

6.3.3. [(diméthoxy-3,4 phényl)-2 éthyl] -1 chlorométhyl-3 pipéridine-(3,*S*)

On refroidit, au bain de glace une solution de 22,2 g (0,07 mole) de chlorhydrate de (-) [(diméthoxy-3,4 phényl)-2 éthyl] -1 pipéridine-(3,*S*)-3-méthanol dans 220 cm$^3$ de $CHCl_3$ et on ajoute à cette température 15,5 cm$^3$ de chlorure de thionyle.

On laisse revenir le mélange à la température ambiante en 1 h 30 puis le porte à la température de reflux pendant 1 h 30.

On refroidit le mélange et l'évapore à sec. On reprend le résidu avec du méthanol et ajoute de l'éther. Le produit cristallise. On obtient un produit fondant à 203°C.

[α] $^{20}{}_D$ = - 9 5° (c=1; $CH_3OH$)

6.3.4. [(diméthoxy-3,4 phényl)-2 éthyl] -1 α-(diméthoxy-3,4 phényl) α-(méthyl-1 éthyl)-pipéridine(-3,*R*)-3-propane nitrile.

On porte à la température du reflux, pendant 10 h, un mélange de 18 g (0,06 mole) du composé obtenu sous 6.3.3., de 16 g (0,07 mole) de α-(diméthoxy-3,4 phényl) α-isopropyl acétonitrile et de 7 cm$^3$ de $NaNH_2$ (à 50 % en suspension dans du toluène) dans 150 cm$^3$ de toluène. On refroidit, reprend avec de l'eau et extrait avec de l'éther. On lave la phase organique avec de l'eau, sèche avec $Na_2SO_4$, filtre et évapore.

Après une purification sur colonne de silice on obtient 17,6 g d'huile.

On prépare l'oxalate dans de l'acétate d'éthyle. On obtient après 2 recristallisations dans de l'alcool d'oxalate de l'énantiomère $A_2$ fondant à 173°C.

[α] $^{20}{}_D$ = - 61,4° (c=1; $CH_3OH$) pour l'oxalate
(Rf=0,45 CCM Merck Art 5719 éluant AcOBu 47, AcOH 28, BuOH 8,5, $H_2O$ 16,5)
[α] $^{20}{}_D$ = - 72° (c=0,5; $CH_3OH$) pour la base.

On évapore le filtrat de cristallisation, reprend le résidu avec de l'eau, alcalinise et extrait avec de l'éther. On lave la phase éthérée avec de l'eau, sèche filtre et évapore. On prépare le chlorhydrate dans un mélange acétone/éther. On obtient ainsi le chlorhydrate de l'énantiomère $B_2$ fondant à 165°C.

[α] $^{20}{}_D$ = - 36° (c=1; $CH_3OH$)
(Rf=0,5 CCM Merck Art 5719 éluant AcOBu 47, AcOH, 28, BuOH 8,5, $H_2O$ 16,5).

**Exemple 7**

Enantiomères $A_1$ et $A_2$ du [(diméthoxy-3,4 phényl) -2 éthyl]-1 α-(diméthoxy-3,4 phényl) α-(méthyl-1 éthyl)-pipéridine-3-propanenitrile (par dédoublement du couple de diastéréoisomères).

On ajoute 5 g (0,0167 mole) de (diméthoxy-3,4 phényl éthyl)-1 chlorométhyl-3 pipéridine en solution dans 4 cm$^3$ de toluène à une suspension de 4 g (0,018 mole) de α-isopropyl α-(diméthoxy-3,4 phényl) acétonitrile, de 8,7 g de potasse en poudre de 0,1 g de bromure de tétrabutylammonium et de 4 cm$^3$ de toluène. On porte le mélange à 50°C pendant 6 heures. On refroidit, le verse sur de l'eau glacée et extrait avec de l'éther. On lave la phase organique avec de l'eau, sèche avec $Na_2SO_4$, filtre et évapore.

On prépare l'oxalate dans du méthanol. On obtient, après recristallisation dans du méthanol, 2,1 g d'oxalate du couple de diastéréoisomères A fondant à 203°C (le fumarate préparé dans un mélange acétone-éther fond à 105°C.).

On solubilise 1 g du couple de diastéréoisomères sous forme de base, 0,8 g d'acide(-)dibenzoyltartrique) dans 10 cm$^3$ d'acétate d'éthyle. On laisse au repos 12 h puis filtre les cristaux obtenus.

Après 2 recristallisations dans de l'acétate d'éthyle on obtient le (-) dibenzoyltartrate de l'énantiomère $A_2$ fondant à 135°C

$[\alpha]^{20}_D = -86,2°$ ($c = 1$; $CH_3OH$)
$[\alpha]^{20}_D = -77°$ ($c = 0,5$; $CH_3OH$) pour la base
On évapore le filtrat de cristallisation, après le traitement habituel pour regénérer la base, on solubilise celle-ci dans 7 $cm^3$ d'acétate d'éthyle et 0,55 g d'acide (+) dibenzoyltartrique et laisse cristalliser. On obtient, après recristallisation dans de l'acétate d'éthyle, le (+) dibenzoyltartrate de l'énantiomère $_1$A fondant à 138° C.
$[\alpha]^{20}_D = +89,4°$ ($c = 1$; $CH_3OH$)
$[\alpha]^{20}_D = +78°$ ($c = 0,5$; $CH_3OH$) pour la base
Le schéma de la synthèse des énantiomères $A_1$, $A_2$, $B_1$ et $B_2$ décrite dans l'exemple 6 est donné dans les pages suivantes 18 et 19.

**Synthèse des énantiomères des composés 1 et 2**

**1ère partie**

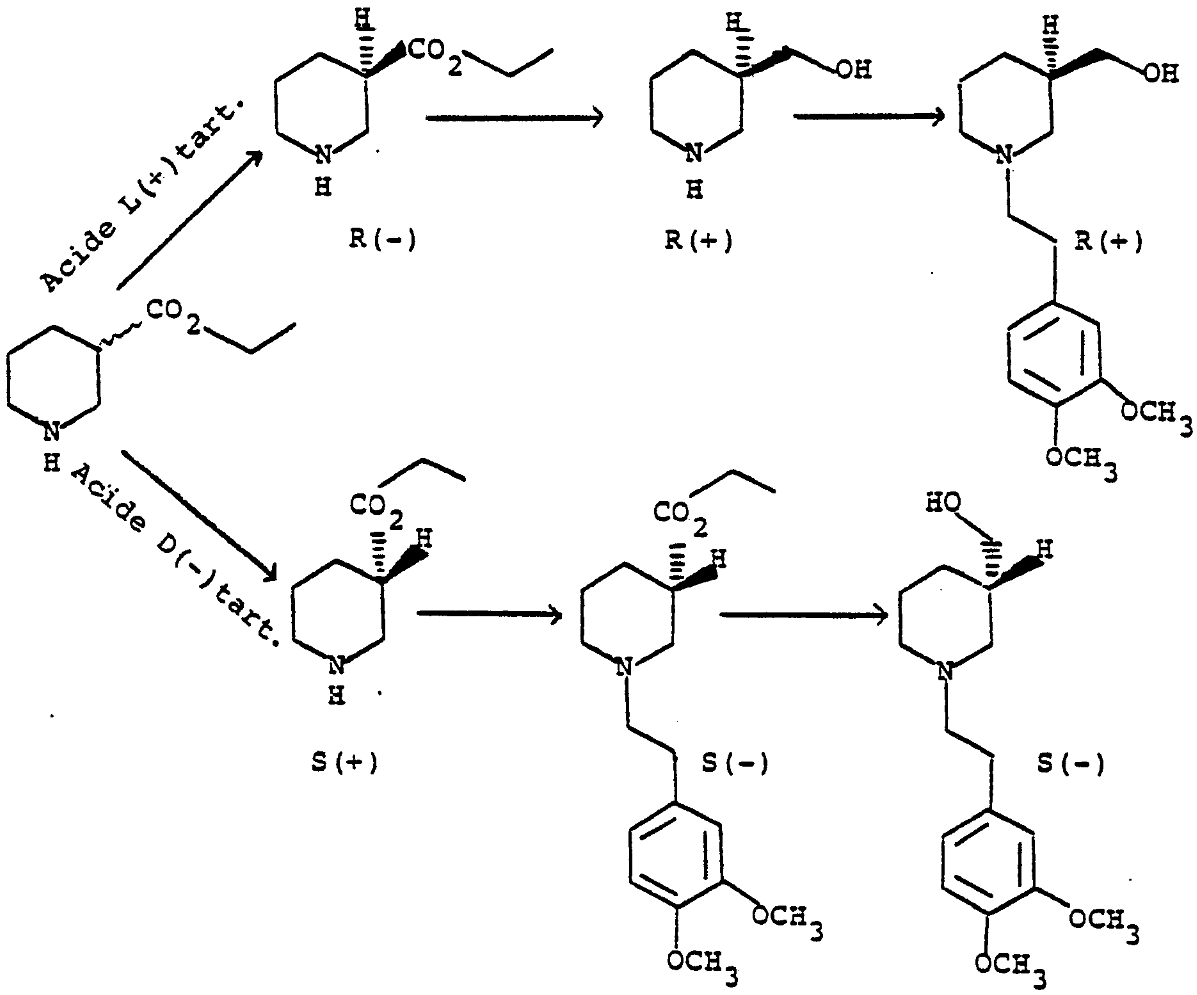

**2e partie**

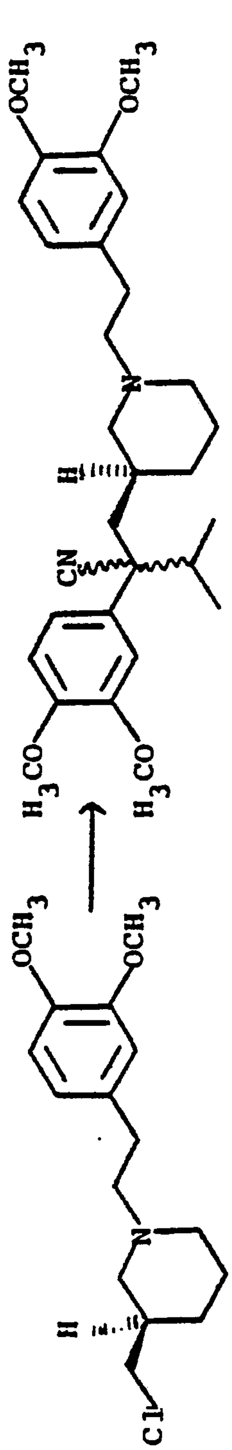

R(+)

+

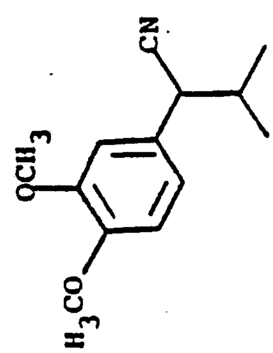

+

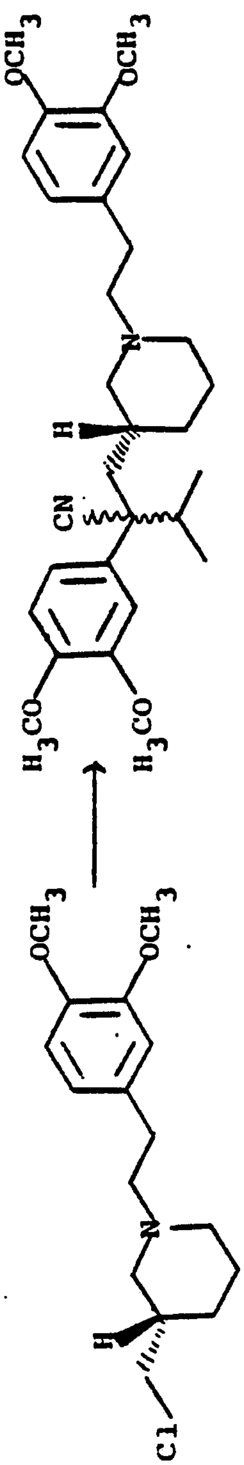

S(–)

Les composés de l'invention préparés à titre d'exemples sont représentés dans le tableau suivant.

**Tableau**

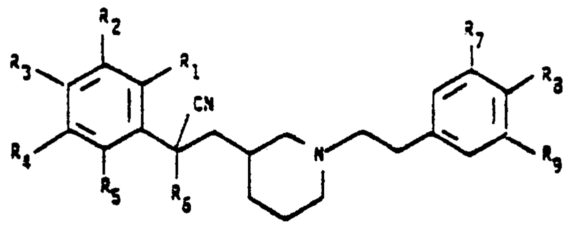

| composé | $R_1$ | $R_2$ | $R_3$ | $R_4$ | $R_5$ | $R_6$ | $R_7$ | $R_8$ | $R_9$ | Sel | F | Dias. |
|---|---|---|---|---|---|---|---|---|---|---|---|---|
| 1 | -H | $-OCH_3$ | $-OCH_3$ | -H | -H | -isopr | $-OCH_3$ | $-OCH_3$ | -H | oxalate | 179° | B |
| 2 | -H | $-OCH_3$ | $-OCH_3$ | -H | -H | -isopr | $-OCH_3$ | $-OCH_3$ | -H | fumarate | 105° | A |
| 3 | -H | $-OCH_3$ | $-OCH_3$ | $-OCH_3$ | -H | -isopr | $-OCH_3$ | $-OCH_3$ | -H | chlorh. | 192° | B |
| 4 | -H | $-OCH_3$ | $-OCH_3$ | $-OCH_3$ | -H | -isopr | $-OCH_3$ | $-OCH_3$ | -H | fumarate | 146° | A |
| 5 | -H | -Cl | -Cl | -H | -H | -isopr | $-OCH_3$ | $-OCH_3$ | -H | chlorh. | 220° | A |
| 6 | -H | -Cl | -Cl | -H | -H | -isopr | $-OCH_3$ | $-OCH_3$ | -H | chlorh. | 201° | B |
| 7 | $-OCH_3$ | $-OCH_3$ | $-OCH_3$ | -H | -H | -isopr | $-OCH_3$ | $-OCH_3$ | -H | oxalate | 200° | A |
| 8 | $-OCH_3$ | $-OCH_3$ | $-OCH_3$ | -H | -H | -isopr | $-OCH_3$ | $-OCH_3$ | -H | chlorh. | 184° | B |
| 9 | -H | $-OCH_3$ | $-OCH_3$ | -H | -H | -H | $-OCH_3$ | $-OCH_3$ | -H | oxalate | 142° | |
| 10 | -H | $-OCH_3$ | $-OCH_3$ | -H | -H | -Et | $-OCH_3$ | $-OCH_3$ | -H | oxalate | 170° | A |
| 11 | -H | $-OCH_3$ | $-OCH_3$ | -H | -H | -Et | $-OCH_3$ | $-OCH_3$ | -H | oxalate | 158° | B |
| 12 | -H | $-OCH_3$ | $-OCH_3$ | -H | -H | 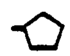 | $-OCH_3$ | $-OCH_3$ | -H | oxalate | 179/180° | A |
| 13 | -H | $-OCH_3$ | $-OCH_3$ | -H | -H | - " - | $-OCH_3$ | $-OCH_3$ | -H | oxalate | 190/191° | B |
| 14 | -H | $-OCH_3$ | $-OCH_3$ | -H | -H | 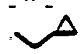 | $-OCH_3$ | $-OCH_3$ | -H | chlorh. | 188° | B |
| 15 | -H | $-OCH_3$ | $-OCH_3$ | -H | -H | - " - | $-OCH_3$ | $-OCH_3$ | -H | oxalate | 175° | A |
| 16 | -H | $-OCH_3$ | $-S-CH_3$ | $-OCH_3$ | -H | -isopr | $-OCH_3$ | $-OCH_3$ | -H | chlorh. | 178° | mél |
| 17 | -H | -Cl | $-OCH_3$ | -H | -H | -isopr | $-OCH_3$ | $-OCH_3$ | -H | fumarate | 155° | B |
| 18 | -H | -Cl | $-OCH_3$ | -H | -H | -isopr | $-OCH_3$ | $-OCH_3$ | -H | fumarate | 184° | A |
| 19 | -H | $-OCH_3$ | -H | $-OCH_3$ | -H | -isopr | $-OCH_3$ | $-OCH_3$ | -H | oxalate | 204° | A |
| 20 | -H | $-OCH_3$ | -H | $-OCH_3$ | -H | -isopr | $-OCH_3$ | $-OCH_3$ | -H | fumarate | 70° | B |
| 21 | -Cl | -Cl | $-OCH_3$ | -H | -H | -isopr | $-OCH_3$ | $-OCH_3$ | -H | chlorh. | 153/154° | A |
| 22 | -Cl | -Cl | $-OCH_3$ | -H | -H | -isopr | $-OCH_3$ | $-OCH_3$ | -H | fumarate | 220/221° | B |
| 23 | -H | $-OCH_3$ | $-OCH_3$ | -H | -H | -isopr | $-OCH_3$ | $-OCH_3$ | -H | oxalate | $\approx$85° | $B_1$ |
| 24 | -H | $-OCH_3$ | $-OCH_3$ | -H | -H | -isopr | $-OCH_3$ | $-OCH_3$ | -H | oxalate | 172° | A |
| 25 | -H | $-OCH_3$ | $-OCH_3$ | -H | -H | -isopr | $-OCH_3$ | $-OCH_3$ | -H | chlorh. | 165° | $B_2$ |
| 26 | -H | $-OCH_3$ | $-OCH_3$ | -H | -H | -isopr | $-OCH_3$ | $-OCH_3$ | -H | oxalate | 173° | $A_2$ |
| 27 | -H | $-OCH_3$ | O | O | -H | -isopr | $-OCH_3$ | $-OCH_3$ | -H | chlorh. | 178/179° | B |
| 28 | -H | $-OCH_3$ | - " - | - " - | -H | -isopr | $-OCH_3$ | $-OCH_3$ | -H | chlorh. | 247° | A |
| 29 | -H | $-OCH_3$ | $-CH_3$ | $-OCH_3$ | -H | -isopr | $-OCH_3$ | $-OCH_3$ | -H | chlorh. | 207° | B |
| 30 | -H | $-OCH_3$ | $-CH_3$ | $-OCH_3$ | -H | -isopr | $-OCH_3$ | $-OCH_3$ | -H | fumarate | 150° | A |
| 31 | -H | $-OCH_3$ | -Cl | -H | -H | -isopr | $-OCH_3$ | $-OCH_3$ | -H | chlorh. | 115/116° | B |
| 32 | -H | $-OCH_3$ | -Cl | -H | -H | -isopr | $-OCH_3$ | $-OCH_3$ | -H | oxalate | 227/228° | A |
| 33 | -H | $-OCH_3$ | -Cl | $-OCH_3$ | -H | -isopr | $-OCH_3$ | $-OCH_3$ | -H | fumarate | 158° | A |
| 34 | -H | $-OCH_3$ | -Cl | $-OCH_3$ | -H | -isopr | $-OCH_3$ | $-OCH_3$ | -H | fumarate | 145° | B |
| 35 | -H | $-OCH_3$ | -H | -H | -H | -isopr | $-OCH_3$ | $-OCH_3$ | -H | oxalate | 166° | B |
| 36 | -H | $-OCH_3$ | -H | -H | -H | -isopr | $-OCH_3$ | $-OCH_3$ | -H | oxalate | 206° | A |
| 37 | -H | $-OCH_3$ | $-OCH_3$ | -H | -H | -isopr | $-OCH_3$ | -H | -H | oxalate | 178° | A |
| 38 | -H | $-OCH_3$ | -O | O- | -H | -isopr | $-OCH_3$ | $-OCH_3$ | -H | chlorh. | 186/188° | B |
| 39 | -H | $-OCH_3$ | - " - | - " - | -H | -isopr | $-OCH_3$ | $-OCH_3$ | -H | oxalate | 222° | A |
| 40 | $-OCH_3$ | -O | O- | -H | -H | -isopr | $-OCH_3$ | $-OCH_3$ | -H | oxalate | 238° | A |

| composé | $R_1$ | $R_2$ | $R_3$ | $R_4$ | $R_5$ | $R_6$ | $R_7$ | $R_8$ | $R_9$ | Sel | F | Dias. |
|---|---|---|---|---|---|---|---|---|---|---|---|---|
| 41 | $-OCH_3$ | -"- | -"- | -H | -H | -isopr | $-OCH_3$ | $-OCH_3$ | -H | chlorh. | 210/211° | B |
| 42 | -H | -H | -O | -O | -H | -isopr | $-OCH_3$ | $-OCH_3$ | -H | chlorh. | 215/220° | A |
| 43 | -H | -H | -"- | -"- | -H | -isopr | $-OCH_3$ | $-OCH_3$ | -H | chlorh. | 172/173° | B |
| 44 | -H | $-OCH_3$ | -H | -H | -H | -isopr | $-OCH_3$ | -H | -H | oxalate | 178° | A |
| 45 | -H | $-OCH_3$ | -H | -H | -H | -isopr | $-OCH_3$ | -H | -H | oxalate | 145° | B |
| 46 | -Cl | $-OCH_3$ | $-OCH_3$ | -H | -H | -isopr | $-OCH_3$ | $-OCH_3$ | -H | oxalate | 197° | A |
| 47 | -H | $-OCH_3$ | $-OCH_3$ | -H | -H | -isopr | $-OCH_3$ | -H | -H | oxalate | 132° | B |
| 48 | -H | $-OCH_3$ | $-OCH_3$ | -H | -H | -isopr | $-OCH_3$ | $-OCH_3$ | -H | oxalate | 157/158° | B |
| 49 | -H | $-OCH_3$ | -H | $-OCH_3$ | -H | -isopr | $-OCH_3$ | -H | -H | oxalate | 210° | A |
| 50 | -H | $-OCH_3$ | -H | $-OCH_3$ | -H | -isopr | $-OCH_3$ | -H | $-OCH_3$ | oxalate | 150° | B |
| 51 | -H | $-OCH_3$ | $-OCH_3$ | -H | -H | -isopr | $-OCH_3$ | -H | $-OCH_3$ | oxalate | 202° | A |
| 52 | O | O | $-OCH_3$ | -H | -H | -isopr | $-OCH_3$ | $-OCH_3$ | -H | fumarate | 179/180° | A |
| 53 | -H | $-OCH_3$ | CME[a] | -H | -H | -isopr | $-OCH_3$ | $-OCH_3$ | -H | oxalate | 184° | A |
| 54 | -H | $-OCH_3$ | CME[a] | -H | -H | -isopr | $-OCH_3$ | $-OCH_3$ | -H | oxalate | 72/74° | B |
| 55 | -H | -H | -F | -H | -H | -isopr | $-OCH_3$ | $-OCH_3$ | -H | oxalate | 172-4° | mél |
| 56 | $-OCH_3$ | $-OCH_3$ | -O | O- | -H | -isopr | $-OCH_3$ | $-OCH_3$ | -H | oxalate | 192° | A |
| 57 | $-OCH_3$ | $-OCH_3$ | -O | O- | -H | -isopr | $-OCH_3$ | $-OCH_3$ | -H | oxalate | 154° | B |
| 58 | $-OCH_3$ | -O | O- | $-OCH_3$ | -H | -isopr | $-OCH_3$ | $-OCH_3$ | -H | oxalate | 209/210° | A |
| 59 | $-OCH_3$ | -O | O- | $-OCH_3$ | -H | -isopr | $-OCH_3$ | $-OCH_3$ | -H | oxalate | 148/150° | B |

CME = cyclopropylméthoxyéthoxy

Le composé N° 1 est décrit dans l'exemple 2
Les composés N° 3 et 4 sont décrits dans l'exemple 3
Le composé N° 6 est décrit dans l'exemple 1
Les composés N° 23 à 26 sont décrits dans l'exemple 6
Les composés N° 40 et 41 sont décrits dans l'exemple 4
Les composés N° 49 et 50 sont décrits dans l'exemple 5

Les composés de l'invention ont été soumis à des essais pharmacologiques montrant leur activité en tant qu'antagoniste du calcium et en tant qu'antihypertenseurs.

1. Le protocole expérimental utilisé est une variante de celui utilisé par Godfraind et Kaba (1969), (blockade or reversal of the contraction induced by calcium and adrenaline in depolarized arterial smooth muscle. Br. J. Pharmac., 36, 549-560).

Les expériences ont été réalisées sur des tronçons d'aorte thoracique de lapin. Les animaux, des "Fauves de Bourgogne" d'un poids moyen de 1,5 kg, sont sacrifiés par dislocation cervicale et exsanguination. L'aorte thoracique est rapidement prélevée et placée dans un milieu de Krebs bicarbonaté oxygéné (95 % $O_2$ + 5 % $CO_2$).

Des tronçons d'aorte de 1 cm de long environ sont préparés et installés dans des cuves à organes de 20 ml contenant de la solution de Krebs bicarbonatée oxygénée (pH 7.4) à 37°C. Deux crochets métalliques en "U" de la longueur des tronçons sont introduits dans la lumière de ceux-ci. L'un des crochets est fixé à la base de la cuve. L'autre, relié à une jauge de contrainte isométrique (Grass FT03), permet l'enregistrement, par l'intermédiaire d'un préamplificateur continu (Grass 7P1), des réponses contractiles des tronçons d'aorte sur un oscillographe à encre (Grass 79B). Cette méthode présente, par rapport aux préparations en spirale ou en anneaux, l'avantage de mieux respecter l'intégrité structurale des vaisseaux et de n'enregistrer que la composante radiale des réponses contractiles qui représente le phénomène intéressant du point de vue fonctionnel (régulation de la pression artérielle). Une tension initiale de 4 g est imposée aux préparations.

De la phénoxybenzamine (1 μM) et du propranolol (1 μM) sont ajoutés aux différents milieu de Krebs afin de supprimer les réponses contractiles liées à l'activation des récepteurs α et β-adrénergiques vasculaires.

Après une heure de stabilisation dans le milieu de Krebs bicarbonaté, la tension imposée aux aortes est ramenée à 2 g. Après une période d'attente de 30 minutes, les préparations sont incubées pendant une dizaine de minutes dans une solution de Krebs bicarbonatée sans calcium en présence d'EDTA (200 μM) et de propranolol (1 μM). Cette solution est alors remplacée par un milieu de Krebs dépolarisant (riche en potassium) sans calcium et contenant du propranolol (1 μM). Après 5 minutes, une concentration unique de 1 mM de calcium est ajoutée à cette solution et une période de stabilisation de 30 minutes est respectée qui permet aux préparations d'atteindre une contraction stable.

Ensuite, des doses cumulatives des composés à tester sont administrées toutes les 30 minutes (temps

généralement nécessaire pour l'obtention d'un plateau) jusqu'à disparition totale de la contraction provoquée par 1 mM de calcium ou bien jusqu'à la concentration maximale de 30 µM de produit. En fin d'expérience, une concentration supramaximale de papavérine (300 µM) est administrée afin de déterminer la décontraction maximale possible de chaque préparation.

Les valeurs absolues (en gramme) de la contraction initiale (après 1 mM de $CaCl_2$) et de la contraction après les différentes concentrations cumulatives de composés vasodilatateurs sont obtenues, pour chaque préparation, par différence avec la contraction minimale observée 30 minutes après l'addition finale de 300 µM de papavérine. Le pourcentage de diminution de la contraction, par rapport à la contraction provoquée par 1 mM de calcium, est calculé pour chaque dose de composé et chaque préparation et ce pourcentage individuel de décontratraction est moyenné $\bar{X}$ ± S.E.M. Les valeurs moyennes obtenues (pondèrées par l'inverse de l'erreur standard à la moyenne), sont analysées à l'aide d'un modèle mathématique de courbe sigmoide et la concentration molaire provoquant 50 % de décontraction de la réponse au calcium ($CE_{50}$) est calculée.

Pour les composés de l'invention la $CE_{50}$ (µM) varie de 0,5 à 1.

2. L'activité antihypertensive des composés de l'invention a été déterminée selon la méthode de Gérald et Tschirky (Arzneim. Forsch. 1968, 18, 1285). La pression systolique est mesurée par captage du pouls au niveau de l'artère caudale. La diminution de la pression est d'environ 30 à 40 % au bout de 2 heures et au bout de 4 heures à la dose de 50 mg/kg administrée per os.

Les composés de l'invention peuvent être utilisés pour le traitement de toutes les maladies où des antagonistes du calcium peuvent être utilisés telles que angine de poitrine, dysrythmie d'origine supraventriculaire, hypertension, cardiomyopathie, protection myocardiaque de patients à risque d'infarctus ou ayant subi un infarctus, arrêt du coeur, accidents cérébrovasculaires, manie, migraines. Ils peuvent également être utilisables comme médicaments, dans le domaine cardiovasculaire, comme antihypertenseurs. Ils sont utilisés dans le traitement de toutes les formes d'hypertension essentielle ou secondaire.

Les composés de l'invention peuvent être présentés sous toute forme appropriée à l'administration orale ou parentérale, en association avec tout excipient approprié, par exemple sous forme de comprimés, gélules, capsules, solutions buvables ou injectables.

La posologie quotidienne peut aller de 10 à 500 mg par voie orale et de 3 à 50 mg par voie parentérale.

**Revendications** pour les Etats contractants BE CH DE FR GB IT LI LU NL SE

1. Dérivés de phénéthyl-1 α-phényl-pipéridine-3-propanenitrile, sous la forme de deux couples de diastéréoisomères et/ou sous la forme d'énantiomères, répondant à la formule

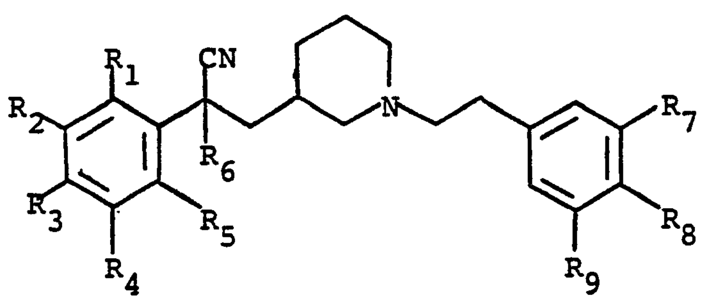

(I)

dans laquelle

· $R_1$, $R_2$, $R_3$, $R_4$ et $R_5$, représentent chacun, indépendamment l'un de l'autre, un atome d'hydrogène, un atome d'halogène, un radical ($C_{1-4}$) alcoxy, un radical ($C_{1-4}$) alkylthio, un radical halogéno-($C_{1-4}$) alkyle, un radical ($C_{1-4}$) alkyle, un radical cyclopropylméthoxyéthoxy, ou deux des radicaux forment ensemble un radical méthylénedioxy ou éthylènedioxy,

· $R_6$ représente un atome d'hydrogène, un radical ($C_{1-4}$) alkyle droit ou ramifié, un radical ($C_{3-6}$) cycloalkyle ou un radical ($C_{3-6}$) cycloalkyl-($C_{1-4}$)alkyle, et

· $R_7$, $R_8$ et $R_9$ représentent chacun, indépendamment l'un de l'autre, un atome d'hydrogène ou un radical méthoxy, ainsi que leurs sels d'addition aux acides pharmaceutiquement acceptables.

2. Dérivés selon la revendication 1, caractérisée par le fait que deux des radicaux $R_1$ à $R_5$ sont différents de H.

3. Dérivés selon la revendication 2, caractérisée par le fait que $R_1$ à $R_5$ représentent chacun, indépendamment l'un de l'autre, un radical méthoxy, méthylthio, méthyle ou cyclo-propylméthoxy-2 éthoxy, ou un atome d'hydrogène, de chlore ou de fluor, ou deux des radicaux $R_1$ à $R_5$ représentent ensemble un radical méthylènedioxy ou éthylènedioxy.

4. Dérivés selon la revendication 3, caractérisée par le fait que $R_6$ est un radical isopropyle, éthyle, cyclopentyle ou cyclopropylméthyle ou un atome d'hydrogène.

5. Dérivés selon la revendication 1, caractérisée par le fait que $R_6$ est le radical isopropyle.

6. Dérivés selon la revendication 1, caractérisée par le fait que deux des radicaux $R_1$ à $R_5$ représentent un radical méthoxy, ou le radical méthylènedioxy ou éthylènedioxy et un autre des radicaux $R_1$ à $R_5$ représente un atome d'hydrogène ou le radical méthoxy, $R_6$ est le radical isopropyle, $R_7$ et $R_8$ représentent chacun un radical méthoxy, et $R_9$ est un atome d'hydrogène.

7. Procédé de préparation des composés selon la revendication 1, procédé caractérisé en ce que l'on fait réagir un composé de formule (II)

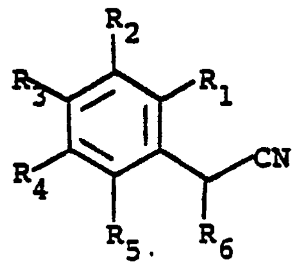

(II)

avec un composé de formule (III)

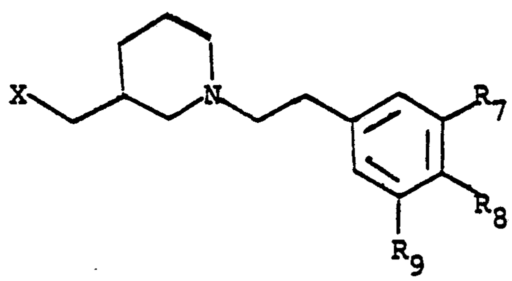

(III)

dans lesquels $R_1$ à $R_9$ ont les significations données dans la revendication 1, et X est un groupe labile, tel qu'un atome d'halogène ou un radical alkylsulfonyle ou arylsulfonyle,

- soit dans du toluène en présence d'amidure de sodium, à une température de 60 à 110° C,
- soit dans un mélange de tétrahydrofuranne et d'hexaméthylphosphorotriamide (HMPT) en présence de diisopropylamidure de lithium à une température de -60 à + 20° C,
- soit par transfert de phase avec une base minérale, telle que la soude et un solvant tel que le toluène ou le chlorure de méthylène en présence d'un ammonium quaternaire tel que le chlorure ou le bromure de tétrabutylammonium, à une température de 20 à 100° C.

8. Médicament caractérisé en ce qu'il contient un composé selon l'une quelconque des revendications 1 à 6.

9. Composition pharmaceutique caractérisée en ce qu'elle contient un composé selon l'une quelconque des revendications 1 à 6 en association avec tout excipient approprié.

**Revendication** pour l'Etat contractant AT

Procédé de préparation de dérivés de phénéthyl-1 α-phényl-pipéridine-3-propanenitrile, sous la forme de deux couples de diastéréoisomères et/ou sous la forme d'énantiomères, répondant à la formule

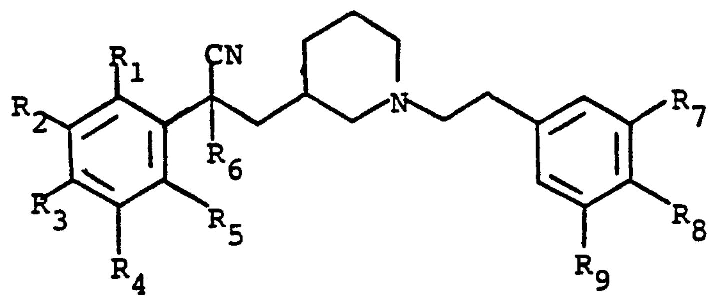

(I)

dans laquelle

· $R_1$, $R_2$, $R_3$, $R_4$ et $R_5$, représentent chacun, indépendamment l'un de l'autre, un atome d'hydrogène, un atome d'halogène, un radical $(C_{1-4})$ alcoxy, un radical $(C_{1-4})$ alkylthio, un radical halogéno-$(C_{1-4})$ alkyle, un radical $(C_{1-4})$ alkyle, un radical cyclopropylméthoxyèthoxy, ou deux des radicaux forment ensemble un radical méthylènedioxy ou éthylènedioxy,

· $R_6$ représente un atome d'hydrogène, un radical $(C_{1-4})$ alkyle droit ou ramifié, un radical $(C_{3-6})$ cycloalkyle ou un radical $(C_{3-6})$ cycloalkyl-$(C_{1-4})$alkyle, et

· $R_7$, $R_8$ et $R_9$ représentent chacun, indépendamment l'un de l'autre, un atome d'hydrogène ou un radical méthoxy, ainsi que leurs sels d'addition aux acides pharmaceutiquement acceptables, procédé caractérisé en ce que l'on fait réagir un composé de formule (II)

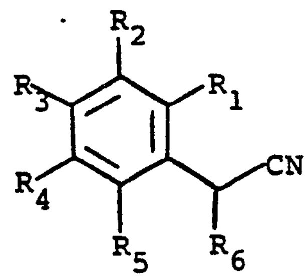

(II)

avec un composé de formule (III)

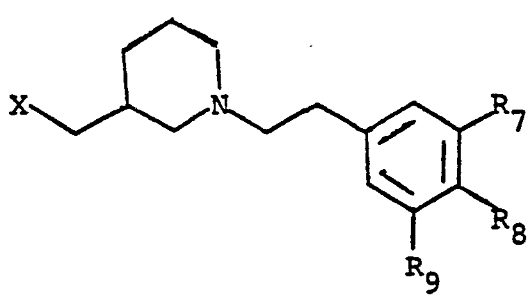

(III)

dans lesquels $R_1$ à $R_9$ ont les significations données dans la revendication 1, et X est un groupe labile, tel qu'un atome d'halogène ou un radical alkylsulfonyle ou arylsulfonyle,

- soit dans du toluène en présence d'amidure de sodium, à une température de 60 à 110°C,
- soit dans un mélange de tétrahydrofuranne et d'hexaméthylphosphorotriamide (HMPT) en présence de diisopropylamidure de lithium à une température de -60 à + 20°C,
- soit par transfert de phase avec une base minérale, telle que la soude et un solvant tel que le toluène ou le chlorure de méthylène en présence d'un ammonium quaternaire tel que le chlorure ou le bromure de tétrabutylammonium, à une température de 20 à 100°C.

**Patentansprüche** für die Vertragsstaaten: BE, CH, DE, FR, GB, IT, LI, LU, NL, SE

1. Derivate des 1-Phenethyl-3-α-phenylpropannitrilo-piperidins, in Form der beiden Diastereoisomerenpaare und/oder in Form der Enantiomeren, der Formel

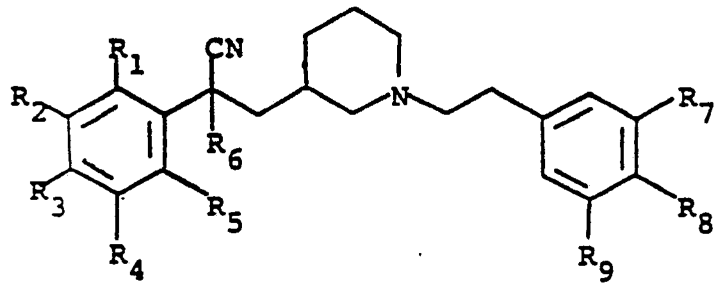

(I)

in welcher $R_1$, $R_2$, $R_3$, $R_4$ und $R_5$ jeweils unabhängig voneinander ein Wasserstoffatom, ein Halogenatom, einen $(C_{1-4})$-Alkoxy-, einen $(C_{1-4})$-Alkylthio-, einen Halogen-$(C_{1-4})$-alkyl-, einen $(C_{1-4})$-alkyl- einen Cyclopropylmethoxyethoxyrest oder zwei dieser Reste gemeinsam einen Methylendioxy- oder Ethylendioxyrest bedeuten, $R_6$ für ein Wasserstoffatom, einen geraden oder verzweigten $(C_{1-4})$-Alkyl-, einen $(C_{3-6})$-Cycloalkyl- oder einen $(C_{3-6})$Cycloalkyl-$(C_{1-4})$-alkylrest steht, und $R_7$, $R_8$ und $R_9$ jeweils unabhängig voneinander ein Wasserstoffatom oder einen Methoxyrest darstellen, sowie deren Additionssalze mit pharmazeutisch verwendbaren Säuren.

2. Derivate nach Anspruch 1, dadurch gekennzeichnet, daß zwei der Reste $R_1$ bis $R_5$ von Wasserstoff verschieden sind.

3. Derivate nach Anspruch 2, dadurch gekennzeichnet, daß $R_1$ bis $R_5$ jeweils unabhängig voneinander für einen Methoxy-, Methylthio-, Methyl- oder 2-Cyclopropylmethoxy-ethoxyrest oder ein Wasserstoff-, Chlor- oder Fluoratom stehen oder zwei der Reste $R_1$ bis $R_5$ gemeinsam einen Methylendioxy- oder Ethylendioxyrest bedeuten.

4. Derivate nach Anspruch 3, dadurch gekennzeichnet, daß $R_6$ einen Isopropyl-, Ethyl-, Cyclopentyl- oder Cyclopropylmethylrest oder ein Wasserstoffatom darstellt.

5. Derivate nach Anspruch 1, dadurch gekennzeichnet, daß $R_6$ für den Isopropylrest steht.

6. Derivate nach Anspruch 1, dadurch gekennzeichnet, daß zwei der Reste $R_1$ bis $R_5$ für einen Methoxy- oder den Methylendioxy- oder Ethylendioxyrest stehen und ein anderer der Reste $R_1$ bis $R_5$ für ein Wasserstoffatom oder den Methoxyrest steht, $R_6$ den Isopropylrest bedeutet, $R_7$ und $R_8$ jeweils für einen Methoxyrest stehen und $R_9$ ein Wasserstoffatom bedeutet.

7. Verfahren zur Herstellung von Verbindungen nach Anspruch 1, dadurch gekennzeichnet, daß man eine Verbindung der Formel (II)

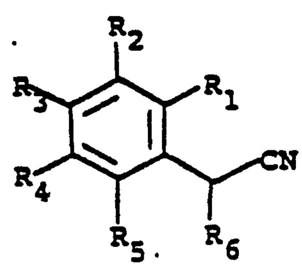

(II)

mit einer Verbindung der Formel (III)

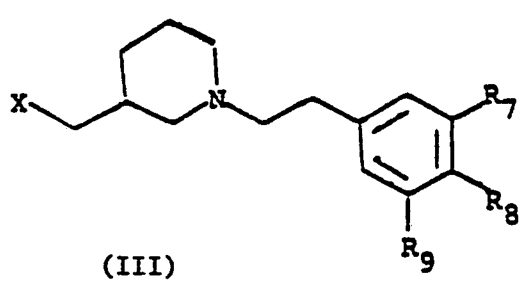

(III)

in welchen $R_1$ bis $R_9$ die in Anspruch 1 angegebene Bedeutung haben und X für eine labile Gruppe, wie für ein Halogenatom oder einen Alkylsulfonyl- oder Arylsulfonylrest steht, reagieren läßt,

- entweder in Toluol in Gegenwart von Natriumamid bei einer Temperatur von 60 bis 110° C oder
- in einer Mischung von Tetrahydrofuran und Hexamethylphosphortriamid (HMPT) in Gegenwart von Lithiumdiisopropylamid bei einer Temperatur von -60 bis + 20° C, oder
- durch Phasentransfer mit einer Mineralbase, wie Soda, und einem Lösungmittel wie Toluol oder Methylenchlorid, in Gegenwart eines quaternären Ammoniumsalzes, wie Tetrabutylammoniumchlorid oder -bromid, bei einer Temperatur von 20 bis 100° C.

8. Heilmittel, dadurch gekennzeichnet, daß es eine Verbindung nach irgendeinem der Ansprüche 1 bis 6 enthält.

9. Pharmazeutische Zusammensetzung, dadurch gekennzeichnet, daß sie eine Verbindung nach irgendeinem der Ansprüche 1 bis 6 gemeinsam mit irgendeinem geeigneten Träger enthält.

**Patentansprüche** für den Vertragsstaat: AT

Verfahren zur Herstellung von Derivaten des 1-Phenethyl-3-α-phenyl-propannitrilo-piperidins, in Form der beiden Diastereoisomerenpaare und/oder in Form der Enantiomeren, der Formel

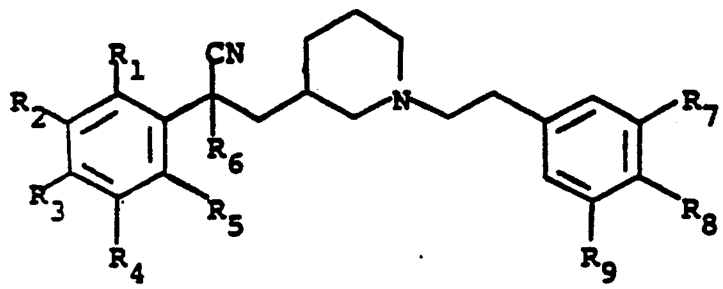

(I)

in welcher $R_1$, $R_2$, $R_3$, $R_4$ und $R_5$ jeweils unabhängig voneinander ein Wasserstoffatom, ein Halogenatom, einen $(C_{1-4})$-Alkoxy-, einen $(C_{1-4})$-Alkylthio-, einen Halogen-$(C_{1-4})$-alkyl-, einen $(C_{1-4})$-alkyl- einen Cyclopropylmethoxyethoxyrest oder zwei dieser Reste gemeinsam einen Methylendioxy- oder Ethylendioxyrest bedeuten, $R_6$ für ein Wasserstoffatom, einen geraden oder verzweigten $(C_{1-4})$-Alkyl-, einen $(C_{3-6})$-Cycloalkyl- oder einen $(C_{3-6})$Cycloalkyl-$(C_{1-4})$-alkylrest steht, und $R_7$, $R_8$ und $R_9$ jeweils unabhängig voneinander ein Wasserstoffatom oder einen Methoxyrest darstellen.

Deren Additionssalzen mit pharmazeutisch verwendbaren Säuren, dadurch gekennzeichnet, daß man eine Verbindung der Formel (II)

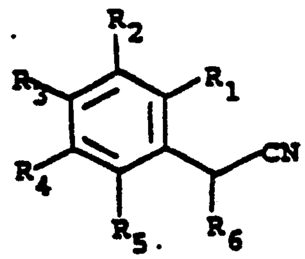

(II)

mit einer Verbindung der Formel (III)

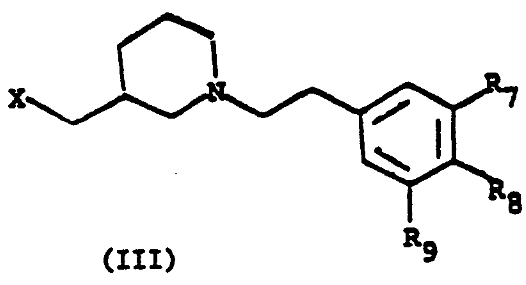

(III)

in welchen $R_1$ bis $R_9$ die Anspruch 1 angegebene Bedeutung haben und X für eine labile Gruppe, wie für ein Halogenatom oder einen Alkylsulfonyl- oder Arylsulfonylrest steht,
reagieren läßt,
- entweder in Toluol in Gegenwart von Natriumamid bei einer Temperatur von 60 bis 110° C oder
- in einer Mischung von Tetrahydrofuran und Hexamethylphosphortriamid (HMPT) in Gegenwart von Lithiumdiisopropylamid bei einer Temperatur von -60 bis + 20° C, oder
- durch Phasentransfer mit einer Mineralbase, wie Soda, und einem Lösungmittel wie Toluol oder Methylenchlorid, in Gegenwart eines quaternären Ammoniumsalzes, wie Tetrabutylammoniumchlorid oder -bromid, bei einer Temperatur von 20 bis 100° C.

**Claims** for the contracting states: BE, CH, DE, FR, GB, IT, LI, LU, NL, SE.

1. 1-Phenetyl-α-phenyl-piperidine-3- propanenitrile derivatives, in the form of two pairs of diastereoisomers and/or in the form of enantiomers, corresponding to the general formula:

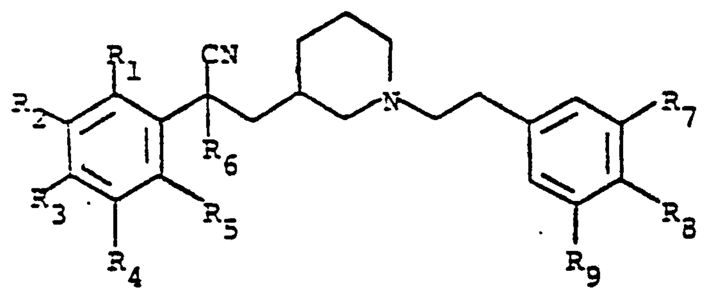

(I)

wherein
$R_1$, $R_2$, $R_3$, $R_4$, and $R_5$ independently of one another each represent a hydrogen atom, a halogen atom, a $(C_{1-4})$alkoxy radical, a $(C_{1-4})$alkylthio radical, a halogeno $(C_{1-4})$alkyl radical, a $(C_{1-4})$alkyl radical or a cyclopropylmethoxyethoxy radical or two of the symbols together form a methylenedioxy or ethylenedioxy radical,
$R_6$ represents a hydrogen atom, a linear or branched $(C_{1-4})$alkyl radical, a $(C_{3-6})$cycloalkyl radical or a $(c_{3-6})$cycloalkyl-$(C_{1-4})$alkyl radical, and
$R_7$, $R_8$ and $R_9$ independently of one another each represent a hydrogen atom or a methoxy radical,
and their pharmaceutically acceptable acid addition salts.

2. Derivatives according to claim 1, characterized in that two of the symbols $R_1$ to $R_5$ are other thatn hydrogen.

3. Derivatives according to claim 2, characterized in that the symbols $R_1$ to $R_5$ independently of one another each represent a methoxy, methylthio, methyl or 2-cyclopropylmethoxy-ethoxy radical or a hydrogen, chlorine or fluorine atom, or two of the symbols $R_1$ to $R_5$ together represent a methylenedioxy or ethylenedioxy radical.

4. Derivatives according to claim 3, characterized in that $R_6$ represents an isopropyl, ethyl, cyclopentyl or cyclopropylmethyl radical or a hydrogen atom.

5. Derivatives according to claim 1, characterized in that $R_6$ represents the isopropyl radical.

6. Derivatives according to claim 1 wherein two of the symbols $R_1$ to $R_5$ represent a methoxy radical or the methylenedioxy or ethylenedioxy radical, another of the symbols $R_1$ to $R_5$ represents a hydrogen atom or the methoxy radical, $R_6$ represents the isopropyl radical, $R_7$ and $R_8$ each represent a methoxy radical and $R_9$ represents a hydrogen atom.

7. Process for the preparation of compounds according to claim 1, characterized in that a compound of formula (II)

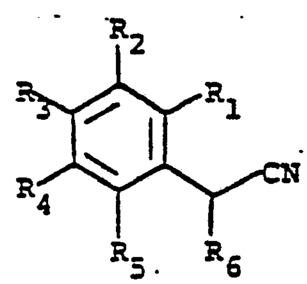

(II)

- is reacted with a compound of formula (III)

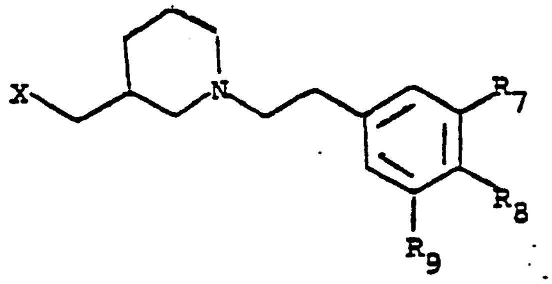

(III)

wherein $R_1$ to $R_9$ are as defined in claim 1 and X represents a labile group such as a halogen atom or an alkylsulphonyl or arylsulphonyl radical,

- either in toluene in the presence of sodium amide at a temperature of from 60° to 110°C,
- or in a mixture of tetrahydrofuran and hexamethylphosphorotriamide (HMPT) in the presence of lithium diisopropylamide at a temperature of from -60 to +20°C,
- or by phase transfer with an inorganic base such as sodium hydroxide, and a solvent such as toluene or methylene chloride, in the presence of a quaternary ammonium compound such as tetrabutylammonium chloride or bromide, at a temperature of from 20° to 100°C.

8. A drug, characterized in that it contains a compound according to anyone of claims 1 to 6.

9. A pharmaceutical composition, characterized in that it contains a compound according to anyone of claims 1 to 6 in combination with any suitable carrier.

**Claim** for the contracting state: AT

A process for the preparation of 1-phenetyl-α-phenyl-piperidine-3- propanenitrile derivatives, in the form of two pairs of diastereoisomers and/or in the form of enantiomers, corresponding to the general formula:

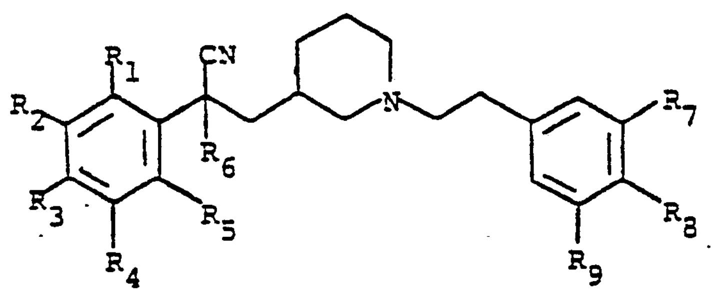

(I)

wherein

$R_1$, $R_2$, $R_3$, $R_4$, and $R_5$ independently of one another each represent a hydrogen atom, a halogen atom, a $(C_{1\text{-}4})$alkoxy radical, a $(C_{1\text{-}4})$alkylthio radical, a halogeno$(C_{1\text{-}4})$alkyl radical, a $(C_{1\text{-}4})$alkyl radical or a cyclopropylmethoxyethoxy radical or two of the symbols together form a methylenedioxy or ethylenedioxy radical,

$R_6$ represents a hydrogen atom, a linear or branched $(C_{1\text{-}4})$alkyl radical, a $(C_{3\text{-}6})$cycloalkyl radical or a $(C_{3\text{-}6})$cycloalkyl-$(C_{1\text{-}4})$alkyl radical, and

$R_7$, $R_8$ and $R_9$ independently of one another each represent a hydrogen atom or a methoxy radical,

and their pharmaceutically acceptable acid addition salts, process characterized in that a compound of formula (II)

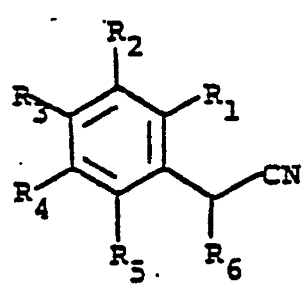

(II)

is reacted with a compound of formula (III)

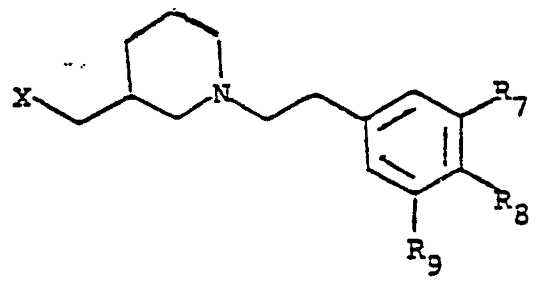

(III)

wherein $R_1$ to $R_9$ are as defined in claim 1 and X represents a labile group such as a halogen atom or an alkylsulphonyl or arylsulphonyl radical,

- either in toluene in the presence of sodium amide at a temperature of from 60° to 110°C,
- or in a mixture of tetrahydrofuran and hexamethylphosphorotriamide (HMPT) in the mresence of lithium diisopropylamide at a temperature of from -60 to +20°C,
- or by phase transfer with an inorganic base such as sodium hydroxide, and a solvent such as toluene or methylene chloride, in the presence of a quaternary ammonium compound such as tetrabutylammonium chloride or bromide, at a temperature of from 20° to 100°C.